# EUROPEAN PATENT APPLICATION

(11) **EP 4 700 041 A1**
(43) Date of publication of application: **25.02.2026**
(21) Application number: 24792965.6
(22) Date of filing: 16.04.2024
(51) Int. Cl.: C07K 7/08, A61K 8/64, A61Q 19/08, A61K 31/728, A61P 17/02, A61K 38/00

(54) **NOVEL PEPTIDE HAVING ACTIVITY OF RETARDING DEGRADATION OF HYALURONIC ACID AND USES THEREOF**

(30) Priority: 17.04.2023 KR 20230050317
(71) Applicant: Caregen Co., Ltd., Anyang-si, Gyeonggi-do 14119 (KR)
(72) Inventor: CHUNG, Yong Ji, Anyang-si, Gyeonggi-do 14119 (KR); KIM, Eun Mi, Anyang-si, Gyeonggi-do 14119 (KR); LEE, Eung Ji, Anyang-si, Gyeonggi-do 14119 (KR); HWANG, Bobyeol, Anyang-si, Gyeonggi-do 14119 (KR); CHO, Mihee, Anyang-si, Gyeonggi-do 14119 (KR); SHIN, Min Lee, Anyang-si, Gyeonggi-do 14119 (KR); LEE, Jaeyeol, Anyang-si, Gyeonggi-do 14119 (KR)
(74) Representative: Handley, Matthew Edward
(86) International application number: PCT/KR2024/005048
(87) International publication number: WO 2024/219779

(57) **Abstract**

with the activity of promoting the activation of skin cells, the peptide of the present invention can be used for skin regeneration or skin condition improvement. In addition, the peptide of the present invention has the activity of retarding or inhibiting the degradation of hyaluronic acid by hyaluronidase and reactive oxygen species, and thus, when used in the preparation of a hyaluronic acid filler, extends the degradation time of the hyaluronic acid filler *in vivo,* thereby enhancing the performance of the filler.

## Description

### TECHNICAL FIELD

### [Cross-reference to Related Applications]

This application claims the benefit of Korean Patent Application No. 10-2023-0050317, filed on April 17, 2023, in the Korean Intellectual Property Office, the disclosure of which is incorporated herein in its entirety by reference.

### [Technical Field]

The present invention relates to a novel peptide having the activity of delaying the degradation of hyaluronic acid and a use thereof.

### BACKGROUND ART

The skin is composed of the epidermis, dermis, and subcutaneous tissue from the outside, and the epidermis is composed of various cells such as keratinocytes made of keratin, melanocytes that form and secrete melanin, Langerhans cells, which are immune-related cells, etc. The dermis is composed of fibrous components and matrix components, and collagen, as a fibrous component, provides strength and tension to the skin and protects it, and accounts for about 90% of the dermis layer. In addition, collagen plays an important role in wound healing, and can promote collagen synthesis in damaged epithelium to enable quick and scar-free recover from wounds. Collagen is synthesized by the action of fibroblasts and degraded by collagenase and elastinase. As we age, the function and number of fibroblasts decrease, which reduces the amount of collagen, which is a fibrous component, synthesized, moisture is lost within skin cells, the structure of the stratum corneum changes, and the function of collagenase increases, which increases the cross-linked form of collagen, thereby reducing smoothness, moisturization, and tightness. In addition to aging, external factors that cause skin aging include pollution, wind, temperature, and ultraviolet rays. In order to prevent skin aging, interest in collagen synthesis promoting substances has increased, and previously known collagen synthesis promoting substances include vitamin C, retinoic acid, proteins derived from animal placenta, betulinic acid, and chlorella extract. However, these substances have limited usage due to safety issues such as irritation and redness when applied to the skin, or their effects are minimal, and thus it is difficult to expect practical effects of improving skin function or healing wounds.

A filler is a substance that is injected into the skin or tissue to increase and expand the volume of soft tissue for the purpose of improving wrinkles, skin sagging, and decreased volume of skin or tissue that occur due to aging. Hyaluronic acid (HA) is a saccharide that forms an essential component of skin and connective tissue, and is a natural polymer polysaccharide composed of repeating disaccharide units of N-acetyl-glucosamine and D-glucuronic acid. The dermis of the skin is filled with a mesh structure of macromolecules called the extracellular matrix, and the extracellular matrix is composed of substances produced by fibroblasts, etc. in the dermis, and is composed of polysaccharides called acidic mucopolysaccharides such as hyaluronic acid and dermatan sulfate, and fibrous proteins such as collagen and elastin. As such, hyaluronic acid is a component that naturally exists in various human tissues, such as the skin, joints, and ocular mucosa, and is widely used as a major filler material because it is easy to use, non-immunogenic, non-carcinogenic, and biocompatible.

It is known that the body contains an enzyme called hyaluronidase that breaks down hyaluronic acid, and this enzyme breaks down hyaluronic acid, and that hyaluronic acid is also degraded by oxidation by free radicals (reactive oxygen species) present in the body. Therefore, even if a hyaluronic acid filler is injected into the body, hyaluronic acid will naturally decompose and disappear. In order to extend the duration of a hyaluronic acid filler, a method has been used in which hyaluronic acid is cross-linked using an appropriate cross-linking agent such as 1,4-Butanediol diglycidyl ether (BDDE) . As such, when a filler is manufactured by cross-linking hyaluronic acid using a cross-linking agent in this way, the filler is protected to some extent from the degradation action of hyaluronidase in the body, and the duration of the filler can be extended. However, the effect is not satisfactory.

Korean Patent No. 10-2369261 discloses a method for producing a core-shell structured hyaluronic acid gel in which a core is manufactured from hyaluronic acid that has been cross-linked primarily by a first cross-linking agent, and a shell is added to the core with hyaluronic acid cross-linked using a second cross-linking agent. Korean Patent No. 10-2225971 discloses a method for producing a hyaluronic acidbased hydrogel using a peptide cross-linking agent. Korean Patent No. 10-2418096 discloses a method for orally administering collagen to maintain the efficacy of a hyaluronic acid filler.

Despite these and other attempts using conventional techniques, a safe and effective technique to delay the degradation of hyaluronic acid fillers has not yet been developed.

### [Prior Art Documents]

### [Patent Documents]

Korea Patent No. 10-2369261
Korea Patent No. 10-2225971
Korea Patent No. 10-2418096

### DISCLOSURE OF THE INVENTION

### TECHNICAL PROBLEM

The present invention has been proposed to solve the above problems, and the purpose of the present invention is to provide a biocompatible peptide that has an activity of delaying the degradation of hyaluronic acid by hyaluronidase and reactive oxygen species and an activity of promoting the activation of skin cells, while having relatively excellent stability even in acids or at high temperatures.

Therefore, an object of the present invention is to provide a novel peptide having an activity of promoting the activation of skin cells and an activity of delaying the degradation of hyaluronic acid.

Another object of the present invention is to provide a cosmetic composition for skin regeneration or skin condition improvement, which contains a peptide having the above-described activity as an active ingredient.

Still another object of the present invention is to provide a pharmaceutical composition for preventing or treating skin diseases, which contains a peptide having the above-described activity as an active ingredient.

Still another object of the present invention is to provide a composition for delaying hyaluronic acid degradation, which contains a peptide having the above-described activity as an active ingredient.

Still another object of the present invention is to provide a filler composition, which contains a peptide having the above-described activity and hyaluronic acid.

### TECHNICAL SOLUTION

An aspect of the present invention provides a peptide containing the amino acid sequence of SEQ ID NO: 1.

Another aspect of the present invention provides a cosmetic composition for skin regeneration or skin condition improvement, which contains the peptide as an active ingredient.

Still another aspect of the present invention provides a pharmaceutical composition for preventing or treating skin diseases, which contains the peptide as an active ingredient.

Still another aspect of the present invention provides a composition for delaying hyaluronic acid degradation, which contains the peptide as an active ingredient.

Still another aspect of the present invention provides a filler composition which contains the peptide and hyaluronic acid.

The present invention is described in detail below.

### Peptides and activities thereof

According to an aspect of the present invention, a peptide containing an amino acid sequence disclosed in SEQ ID NO: 1 is provided.

[Amino acid sequence of SEQ ID NO: 1]

KIWNLEPSRNGT (H2N-Lys-Ile-Trp-Asn-Leu-Glu-Pro-Ser-
Arg-Asn-Gly-Thr-COOH)

The term "peptide" as used herein means a linear molecule formed by amino acid residues joining together by peptide bonds.

The peptide containing the amino acid sequence of SEQ ID NO: 1 of the present invention may be used without modification, but a mutant or fragment of an amino acid having a different sequence by deletion, insertion, substitution, or a combination thereof of amino acid residues may be used within a range that does not affect the original activity of the peptide, for example, the activity of inhibiting or delaying the degradation of hyaluronic acid by hyaluronidase or a reactive oxygen species species.

The peptide of the present invention may be modified by phosphorylation, sulfation, acrylation, glycosylation, methylation, farnesylation, etc., within a range that does not change its activity.

The peptide of the present invention includes a peptide containing an amino acid sequence substantially identical to a peptide containing an amino acid sequence of SEQ ID NO: 1, and a variant or active fragment thereof. The substantially identical amino acid sequence means an amino acid sequence having a sequence identity of 75% or more, for example, 80% or more, 85% or more, 90% or more, 95% or more, or 97% or more, to the amino acid sequence of SEQ ID NO: 1. In addition, the peptide may additionally include a targeting sequence, a tag, a labeled residue, an amino acid sequence prepared for a specific purpose of increasing half-life or peptide stability.

The peptide of the present invention may be one in which a portion of the amino acid sequence is selected and N-terminal and/or C-terminal modifications are induced to increase its activity. Through such N-terminal and/or C-terminal modifications, the stability of the peptide of the present invention may be significantly improved, and for example, the half-life of the peptide when administered *in vivo* may be increased. The term "stability" includes not only stability *in vivo* that protects the peptide of the present invention from attack by protein cleavage enzymes *in vivo,* but also storage stability (e.g., room temperature storage stability).

The N-terminal modification may be a modification in which a protecting group selected from the group consisting of an acetyl group, a fluoreonylmethoxycarbonyl group, a formyl group, a palmitoyl group, a myristyl group, a stearyl group, and polyethylene glycol (PEG) is bonded to the N-terminus of the peptide. The C-terminal modification may be a modification in which a hydroxyl group (-OH), an amino group (-NH₂), an azide (-NHNH₂), *etc.* is bonded to the C-terminus of the peptide, but is not limited thereto.

The peptide of the present invention may be prepared by various methods widely known in the art to which the present invention pertains. For example, the peptide of the present invention may be prepared by chemical synthesis methods known in the art, particularly solid-phase synthesis techniques (Merrifield, J. Amer. Chem. Soc. 85:2149-54(1963) ; Stewart, et al., Solid Phase Peptide Synthesis, 2nd. ed., Pierce Chem. Co.: Rockford, 111(1984)) or liquid-phase synthesis techniques (U.S. Patent No. 5,516,891).

The peptide of the present invention has the activity of activating skin cells.

In an embodiment, the peptide of the present invention has the activity of promoting the proliferation of fibroblasts or keratinocytes.

In an embodiment, the peptide of the present invention has the activity of promoting the expression of collagen or hyaluronic acid.

In an embodiment, the peptide of the present invention has the activity of promoting the expression of collagen or hyaluronic acid in fibroblasts or keratinocytes.

In an embodiment, the peptide of the present invention has the activity of delaying or inhibiting the degradation of hyaluronic acid.

In an embodiment, the degradation of hyaluronic acid may be degradation by hyaluronidase or degradation by reactive oxygen species.

As described above, the peptide of the present invention has an activity of delaying or inhibiting the degradation of hyaluronic acid by hyaluronidase or reactive oxygen species; therefore, when used together with a filler made of hyaluronic acid, the peptide exhibits the effect of extending the period of existence of the hyaluronic acid filler in the body, thereby improving the performance of the filler.

In addition, the peptide of the present invention has stability under pH and temperature changes. As demonstrated in the examples described below, the peptide of the present invention exhibits the same stability in the range of pH 4 to pH 10 and the temperature range of 25°C to 50°C, and thus has stability under pH and temperature changes, thereby having excellent biostability.

### Composition containing peptide

In another aspect of the present invention, the present invention provides a composition for skin regeneration or skin condition improvement, which contains a peptide containing an amino acid sequence of SEQ ID NO: 1 as an active ingredient.

In still another aspect of the present invention, the present invention provides a cosmetic composition for skin regeneration or skin condition improvement, which contains a peptide containing an amino acid sequence of SEQ ID NO: 1 as an active ingredient.

The term "improvement" as used herein means any action that improves or benefits the symptoms of a disease or disorder.

In this specification, the improvement of skin condition comprehensively refers to the process of treating, reducing, or alleviating damage to the skin caused by intrinsic or extrinsic factors of the skin, effects thereof, etc.

In an embodiment, the improvement of the skin condition may be an increase in skin elasticity, an improvement in skin wrinkles, an increase in skin moisturization, an inhibition of skin aging, a recovery from skin damage caused by ultraviolet rays, a protection of the skin from ultraviolet rays, or an improvement of skin wounds.

In an embodiment, the peptide containing the amino acid sequence of SEQ ID NO: 1 contained as an active ingredient in the cosmetic composition promotes the expression of collagen or hyaluronic acid in skin cells, for example, fibroblasts or keratinocytes.

In the present invention, the "improvement of skin wrinkles", "promotion of skin elasticity", "inhibition of skin aging", "recovery of skin damage caused by ultraviolet rays", "protection of skin from ultraviolet rays", or "improvement of skin wounds" may be induced by any action that increases the total amount of collagen in the skin, including promotion of collagen synthesis in skin cells, etc. The skin cells may be, for example, fibroblasts or keratinocytes.

The "inhibition of skin aging" may mean inhibiting functional deterioration in the skin, such as wrinkles, sagging skin, loss of elasticity, etc. The skin aging may be photoaging, for example, skin aging caused by ultraviolet rays.

The cosmetic composition of the present invention may further contain a cosmetically acceptable carrier in addition to a peptide containing the amino acid sequence of SEQ ID NO: 1.

The cosmetic composition may be prepared into any formulation commonly prepared in the technical field to which the present invention belongs, and may be a topical skin preparation. For example, it may be formulated into a solution, suspension, emulsion, paste, gel, cream, lotion, powder, soap, surfactant-containing cleansing, oil, powder foundation, emulsion foundation, wax foundation, spray, etc., but is not limited thereto.

The cosmetic composition may be prepared in various forms, such as a solution (e.g., a softening toner, a nourishing toner, a nourishing cream, a massage cream, an essence, an eye cream, a cleansing cream, a cleansing foam, a cleansing water, a pack, a spray, a powder, a hair tonic, a hair cream, a hair lotion, a hair shampoo, a hair rinse, a hair conditioner, a hair spray, a hair aerosol, a pomade, a gel, etc.), sol-gel, emulsion, oil, wax, aerosol, etc., but is not limited thereto.

The cosmetic composition of the present invention may contain other additives such as excipients and carriers, and it is possible to apply and mix as much as necessary the common ingredients mixed in general skin cosmetics.

When the formulation of the cosmetic composition is in the form of a paste, cream, or gel, it is possible to use animal oil, vegetable oil, wax, paraffin, starch, tragacanth, cellulose derivatives, polyethylene glycol, silicone, bentonite, silica, talc, zinc oxide, etc. as a carrier component.

When the formulation of the cosmetic composition is in the form of a powder or spray, it is possible to contain lactose, talc, silica, aluminum hydroxide, calcium silicate, or polyamide powder as a carrier component, and particularly in the case of a spray, it is possible to additionally contain a propellant such as chlorofluorohydrocarbon, propane/butane, or dimethyl ether, but is not limited thereto.

When the formulation type of the cosmetic composition is a solution or emulsion, it is possible to use a solvent, a solubilizer, or an emulsifier as a carrier component, and examples thereof may include water, ethanol, isopropanol, ethyl carbonate, ethyl acetate, benzyl alcohol, benzyl benzoate, propylene glycol, 1,3-butyl glycol oil, glycerol aliphatic ester, polyethylene glycol, or fatty acid ester of sorbitan etc.

When the formulation type of the cosmetic composition is in the form of a suspension, it is possible to use liquid diluents (e.g., water, ethanol, propylene glycol, etc.), suspending agents (e.g., ethoxylated isostearyl alcohol, polyoxyethyl sorbitol ester, polyoxyethylene sorbitan ester, etc.), microcrystalline cellulose, aluminum metahydroxide, bentonite, agar, tragacanth, etc. may be used as carrier components.

When the formulation of the cosmetic composition is a surfactant-containing cleansing, it is possible to use, as the carrier component, fatty alcohol sulfate, fatty alcohol ether sulfate, sulfosuccinic acid monoester, isethionate, imidazolinium derivatives, methyl taurate, sarcosinate, fatty acid amide ether sulfate, alkyl amidobetaine, fatty alcohol, fatty acid glyceride, fatty acid diethanolamide, vegetable oil, lanolin derivatives, ethoxylated glycerol fatty acid ester, *etc.*

When the formulation of the cosmetic composition is a hair shampoo, base components for constituting a shampoo, such as a thickener, a surfactant, a viscosity regulator, a moisturizer, a pH regulator, a preservative, and essential oils, may be mixed with the peptide of the present invention. CDE may be used as the thickener, LES, which is an anionic surfactant, and coco betaine, which is an amphoteric surfactant, may be used as the surfactant, polyquat may be used as the viscosity regulator, glycerin may be used as the moisturizer, and citric acid or sodium hydroxide may be used as the pH regulator. A grapefruit extract, etc. may be used as the preservative, and in addition, essential oils (e.g., cedarwood, peppermint, rosemary, etc.), silk amino acids, pentaol, or vitamin E may be added.

The ingredients contained in the cosmetic composition may further include, in addition to the peptide of the present invention and the carrier components as active ingredients, ingredients commonly used in cosmetic compositions, such as conventional auxiliary agents (e.g., antioxidants, stabilizers, solubilizers, vitamins, pigments, and fragrances), but are not limited thereto.

The cosmetic composition of the present invention may contain a cosmetically effective amount of a peptide containing the amino acid sequence of SEQ ID NO: 1.

The cosmetically effective amount means an amount sufficient to achieve the effect of skin regeneration or skin condition improvement of the cosmetic composition.

The peptide of the present invention may be contained in the cosmetic composition described above at a concentration of 0.01 µM to 1000 µM, specifically, the peptide of the present invention may be contained in the cosmetic composition described above at a concentration of 0.01 µM to 1000 µM; 0.05 µM to 800 µM, 0.05 µM to 700 µM, 0.05 µM to 600 µM, 0.05 µM to 500 µM, 0.05 µM to 300 µM, 0.05 µM to 200 µM; 0.1 µM to 800 µM, 0.1 µM to 700 µM, 0.1 µM to 600 µM, 0.1 µM to 500 µM, 0.1 µM to 300 µM, 0.1 µM to 200 µM; 1 µM to 800 µM, 1 µM to 700 µM, 1 µM to 600 µM, 1 µM to 500 µM, 1 µM to 300 µM, 1 µM to 200 µM; 5 µM to 800 µM, 5 µM to 700 µM, 5 µM to 600 µM, 5 µM to 500 µM, 5 µM to 300 µM, or 5 µM to 200 µM, but is not limited to.

In still another aspect of the present invention, the present invention provides a pharmaceutical composition for preventing or treating skin diseases, which contains a peptide containing an amino acid sequence of SEQ ID NO: 1 as an active ingredient.

In an embodiment, the skin condition may be a wound or scar on the skin.

In an embodiment, the peptide, which contains the amino acid sequence of SEQ ID NO: 1 contained as an active ingredient in the pharmaceutical composition, promotes the expression of collagen or hyaluronic acid in skin cells, for example, fibroblasts or keratinocytes.

The pharmaceutical composition of the present invention may contain a therapeutically effective amount of the peptide of the present invention.

The term "therapeutically effective amount" means an amount sufficient for the peptide, which is an active ingredient of the pharmaceutical composition of the present invention, to achieve its activity or efficacy, for example, an amount sufficient to achieve efficacy in treating or preventing skin diseases.

The term "prevention" as used herein means reducing the risk of developing a disease or disorder, and means any action that inhibits or delays the onset of a disease by preventing the disease or one or more of its clinical symptoms from progressing.

The term "treatment" as used herein means alleviating a disease or disorder, and includes any action that improves or beneficially alters the symptoms of a disease by arresting or reducing the progression of the disease or one or more of its clinical symptoms.

In the present invention, prevention or treatment of skin disease may be to remove the cause of the skin disease or to inhibit the progression of the skin disease.

The pharmaceutical composition of the present invention may contain a peptide containing an amino acid sequence of SEQ ID NO: 1 and a pharmaceutically acceptable carrier.

The pharmaceutically acceptable carriers mentioned above are those commonly used in the preparation of formulations and include, but are not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, etc.

The pharmaceutical composition of the present invention may additionally contain, in addition to the above components, a lubricant, a humectant, a sweetener, a flavoring agent, an emulsifier, a suspending agent, a preservative, etc., but is not limited thereto.

Suitable pharmaceutically acceptable carriers and formulations are described in detail in Remington: The Science and Practice of Pharmacy, (19th ed., 1995, Williams & Wilkins).

The pharmaceutical composition of the present invention may be administered by any suitable route for treating skin diseases, and for example, may be administered orally or parenterally, and in the case of parenteral administration, it may be administered by intravenous injection, subcutaneous injection, intramuscular injection, intraperitoneal injection, topical administration, transdermal administration, etc. Since the pharmaceutical composition of the present invention has an activity for preventing or treating skin diseases, it may be applied by topical administration such as application to the skin.

The dose of the pharmaceutical composition may be, but is not limited to, 0.0001 µg to 100 mg, 0.001 µg to 100 mg, 0.01 µg to 100 mg, 0.1 µg to 100 mg, or 1.0 µg to 1000 mg per day, and may be prescribed in various ways depending on factors such as the formulation method, administration method, patient's age, weight, sex, pathological condition, food, administration time, administration route, excretion rate, and reaction sensitivity.

The pharmaceutical composition of the present invention may be prepared in a unit dose form or may be prepared by placing it in a multi-dose container by formulating it using a pharmaceutically acceptable carrier and/or excipient according to a method that may be easily performed by a person having ordinary skill in the art to which the present invention pertains. In particular, the formulation may be in the form of a solution, suspension, or emulsion in an oil or aqueous medium, or in the form of an extract, powder, granules, tablets, or capsules, and may additionally contain a dispersant or stabilizer.

The pharmaceutical composition of the present invention may be a topical skin preparation. The topical skin preparation is a preparation in a form that can be applied to the outside of the skin and used. When the pharmaceutical composition of the present invention is used as a topical skin preparation, it may be applied to the skin, specifically, to a skin area where a skin disease has occurred. The topical skin preparation may be a cream, a gel, an ointment, a skin emulsifier, a skin suspension, a transdermal delivery patch, a drug-containing bandage, a lotion, or a combination thereof. The topical skin preparation may be appropriately mixed with ingredients commonly used in topical skin preparations such as cosmetics or medicines, for example, aqueous ingredients, oily ingredients, powder ingredients, alcohols, moisturizers, thickeners, ultraviolet absorbers, whitening agents, preservatives, antioxidants, surfactants, fragrances, colorants, various skin nutrients, or combinations thereof, as needed. The topical skin preparation may also appropriately contain metal sequestrants such as disodium edetate, trisodium edetate, sodium citrate, sodium polyphosphate, sodium metaphosphate, and gluconic acid; caffeine, tannin, a licorice extract, glabridin, various herbal medicine, tocopheryl acetate, glycyrrhizic acid, tranexamic acid and derivatives, or salts thereof; and sacchardies such as vitamin C, magnesium ascorbic acid phosphate, ascorbic acid glucoside, arbutin, kojic acid, glucose, fructose, and trehalose.

Still another aspect of the present invention provides a composition for delaying the degradation of hyaluronic acid, which contains a peptide having an amino acid sequence of SEQ ID NO: 1 as an active ingredient.

Still another aspect of the present invention provides a use of a peptide containing an amino acid sequence of SEQ ID NO: 1 for delaying the degradation of hyaluronic acid.

In an embodiment, the degradation of hyaluronic acid may be degradation by hyaluronidase or degradation by reactive oxygen species.

In the present invention, "delay" of hyaluronic acid degradation is used as a meaning equivalent to "inhibition" or "suppression" of hyaluronic acid degradation.

Still another aspect of the present invention provides a filler composition, which contains a peptide containing an amino acid sequence of SEQ ID NO: 1 and hyaluronic acid.

In the present invention, hyaluronic acid (HA) refers to a biopolymer represented by the following Formula 1 in which repeating units composed of N-acetyl-D-glucosamine and D-glucuronic acid are linearly connected.

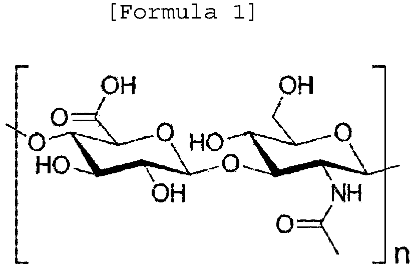

Hyaluronic acid is found in large quantities in the vitreous humor of the eye, synovial fluid of joints, and rooster combs, and due to its excellent biocompatibility, it is widely used in medical and medical device applications such as ophthalmic surgical adjuvants, joint function improving agents, drug delivery materials, eye drops, and wrinkle improving agents, use in cosmetics.

In an embodiment, the hyaluronic acid contained in the filler composition of the present invention may include a salt thereof in addition to hyaluronic acid. The salt of hyaluronic acid includes inorganic salts such as sodium hyaluronate, potassium hyaluronate, calcium hyaluronate, magnesium hyaluronate, zinc hyaluronate, and cobalt hyaluronate, and organic salts such as tetrabutylammonium hyaluronate, but is not limited thereto.

In an embodiment, the hyaluronic acid or a salt thereof may be hyaluronic acid cross-linked by a suitable cross-linking agent.

In an embodiment, the cross-linked hyaluronic acid may be one cross-linked with one or more cross-linking agents selected from the group consisting of 1,4-butane diol diglycidyl ether (BDDE), polyethylene glycol (PEG), divinyl sulfone (DVS), poly(ethylene glycol) diglycidyl ether (PEGDGE), 1,6-hexanediol diglycidyl ether, propylene glycol diglycidyl ether, poly(propylene glycol) diglycidyl ether, and poly(tetramethylene glycol) diglycidyl ether.

In the filler composition of the present invention, the content of the peptide including the amino acid sequence of SEQ ID NO: 1 may be 0.001-0.5 wt%, 0.002-0.5 wt%, 0.003-0.5 wt%, 0.004-0.5 wt%, 0.005-0.5 wt%, 0.001-0.4 wt%, 0.002-0.4 wt%, 0.003-0.4 wt%, 0.004-0.4 wt%, 0.005-0.4 wt%, 0.001-0.3 wt%, 0.002-0.3 wt%, 0.003-0.3 wt%, 0.004-0.3 wt%, 0.005-0.3 wt%, 0.001-0.2 wt%, 0.002-0.2 wt%, 0.003-0.2 wt%, 0.004-0.2 wt%, 0.005-0.2 wt% relative to the total composition, but is not limited thereto.

In the filler composition of the present invention, the content of hyaluronic acid may be 0.001-0.5 wt%, 0.002-0.4 wt%, 0.003-0.3 wt%, 0.004-0.2 wt%, or 0.005-0.1 wt% relative to the total composition, but is not limited thereto.

In an embodiment, the peptide containing the amino acid sequence of SEQ ID NO: 1 and hyaluronic acid in the filler composition of the present invention may be in a simply mixed state.

The term "filler" used in the present invention is used broadly to refer to a material or composition designed to add volume to a soft tissue deficient area, and also includes the meaning of a soft tissue filler or dermal filler.

The term "soft tissue" used the present invention generally refers to tissue that connects, supports, or surrounds other structures and organs of the body. Such soft tissues include, for example, muscles, tendons, vocal cords, endothelial tissue, fibrous tissue, fat, blood vessels, nerves, and synovial tissue.

In an embodiment, the filler composition may optionally include, but is not limited to, buffering agents, preservatives, isotonic agents, antioxidants, emulsifiers, humectants, etc., and may include other pharmaceutically acceptable ingredients.

The buffering agent may include one or more selected from the group consisting of citric acid, sodium monohydrogen phosphate, sodium dihydrogen phosphate, acetic acid, diethyl barbituric acid, sodium acetate, tris(hydroxymethyl) methylamino) propanesulfonic acid (TAPS), 2-bis (2-hydroxyethyl) amino) acetic acid (Bicine), tris (hydroxymethyl) ammonium methane (Tris), N-(2-hydroxy-1,1-bis (hydroxymethyl)ethyl)glycine (Tricine), 4-(2-hydroxyethyl)-1-piperazine ethanesulfonic acid (HEPES), 2-[[1,3-dihydroxy-2-(hydroxymethyl)propan-2-yl]amino] methanesulfonic acid (TES), and piperazine-N, N'-bis (2-ethanesulfonic acid (PIPES), but is not limited thereto.

The isotonic agent may be used without limitation as long as it is used in the preparation of a hyaluronic acid filler, and may be contained in the buffer solution. As a preferred isotonic agent, sodium chloride may be used.

In an embodiment, the filler composition of the present invention may further contain other substances or combinations of substances that provide a beneficial effect when administered to a subject, including but not limited to, antioxidants, anti-itch agents, anti-cellulite agents, antiscar agents, anti-inflammatory agents, anesthetics, irritantrelaxing agents, vasoconstrictors, vasodilators, antihemorrhagic agents such as hemostatic agents or antifibrinolytic agents, exfoliants, elasticizers, anti-acne agents, colorants, anti-pigmentation agents, or moisturizers.

In an embodiment, the filler composition may be used for improving wrinkles and reducing tissue volume in soft tissues selected from the group consisting of the neck, chest, buttocks, arms, armpits, hands, legs, and feet, or for treating wounds, scars, or stretch marks.

In an embodiment, the filler composition may be a cosmetic composition, and preferably a cosmetic composition for improving wrinkles or reducing tissue volume of soft tissue selected from the group consisting of the neck, chest, buttocks, arms, armpits, hands, legs, and feet.

In an embodiment, the filler composition may be a pharmaceutical composition, preferably a pharmaceutical composition for use in treating or preventing wounds, scars or stretch marks.

When the filler composition is in the form of a cosmetic composition or pharmaceutical composition, the technical contents and technical features described with respect to the cosmetic composition and the pharmaceutical composition in the composition items described above are applied as they are to the filler composition of the present invention, and thus are not described repeatedly in order to avoid excessive complexity of the specification.

The filler composition of the present invention may be administered in a dosage form suitable for the above-mentioned use, and preferably in the form of an injection.

The filler composition of the present invention may undergo a sterilization process and may be quantitatively filled, sealed, and sterilized in an appropriate container, such as a syringe.

### Uses of Peptides

In still another aspect of the present invention, the present invention provides a use of the peptide containing an amino acid sequence of SEQ ID NO: 1 for skin regeneration or skin condition improvement.

In still another aspect of the present invention, the present invention provides a use of the peptide containing an amino acid sequence of SEQ ID NO: 1 in the preparation of a cosmetic for skin regeneration or skin condition improvement.

In still another aspect of the present invention, the present invention provides a use of the peptide containing an amino acid sequence of SEQ ID NO: 1 for the treatment of a skin disease.

In still another aspect of the present invention, the present invention provides a use of the peptide containing an amino acid sequence of SEQ ID NO: 1 in the preparation of a medicament for treating a skin disease.

In still another aspect of the present invention, the present invention provides a use of a peptide containing an amino acid sequence of SEQ ID NO: 1 for delaying the degradation of hyaluronic acid degradation.

Since the technical contents and technical features of the present invention described in the peptide and composition of the present invention are all applied to the uses of the peptide of the present invention described above in the same manner, these technical contents and technical features will not be described repeatedly in order to avoid excessive complexity of the specification.

### ADVANTAGEOUS EFFECTS

The peptide of the present invention has an activity of promoting skin cell activation, and thus can be used for skin regeneration or skin condition improvement. In addition, the peptide of the present invention has an activity of delaying or inhibiting the degradation of hyaluronic acid by hyaluronidase and reactive oxygen species; therefore, when used together in the preparation of hyaluronic acid filler, it has the effect of improving the performance of the filler by extending the time for the hyaluronic acid filler to be degraded in the body.

However, the effects of the present invention are not limited to the effects mentioned above, and other effects not mentioned will be clearly understood by those skilled in the art from the description below.

### BRIEF DESCRIPTION OF THE DRAWINGS

FIG. 1 is an experimental result showing the effect of the peptide of the present invention on promoting the proliferation of NIH3T3 cells (mouse fibroblast) and HaCaT cells (human keratinocyte cell line).
FIG. 2 is an experimental result showing the effect of the peptide of the present invention on promoting the expression of collagen and hyaluronic acid (HA) in NIH3T3 cells (mouse fibroblast) and HaCaT cells (human keratinocyte cell line).
FIG. 3 is an experimental result showing the effect of the peptide of the present invention on delaying the degradation of hyaluronic acid by hyaluronidase in a hyaluronic acid solution.
FIG. 4 is an experimental result showing the effect of the peptide of the present invention on delaying the degradation of hyaluronic acid by hyaluronidase in a BDDE-cross-linked hyaluronic acid filler.
FIG. 5 is an experimental result showing the effect of the peptide of the present invention on delaying the degradation of hyaluronic acid by reactive oxygen species (hydrogen peroxide) in a hyaluronic acid solution.
FIG. 6 is an experimental result showing the effect of the peptide of the present invention on delaying the degradation of hyaluronic acid by reactive oxygen species (hydrogen peroxide) in a BDDE-cross-linked hyaluronic acid filler.
FIG. 7a is an experimental result showing that the peptide of the present invention is stable even at a high temperature of 50°C.
FIG. 7b is an experimental result showing that the peptide of the present invention is stable even in the pH range of 4 to 10.

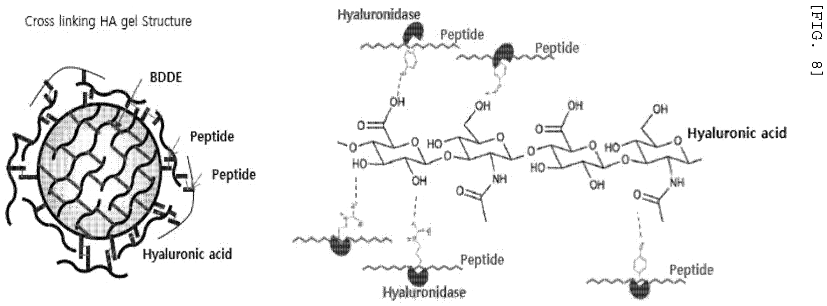

FIG. 8 is a schematic diagram showing the action of the peptide of the present invention to delay the degradation of hyaluronic acid by hyaluronidase.

### MODE FOR CARRYING OUT THE INVENTION

Hereinafter, the present invention will be described in detail by way of examples. However, the following examples are intended to specifically illustrate the present invention, and the content of the present invention is not limited by the following examples.

### Preparation Example 1: Preparation of peptides and peptide complexes

Using an automatic peptide synthesizer (CEM Liberty, CEM Corporation, USA), peptides having an amino acid sequences of SEQ ID NO: 1 as shown in Table 1 below were synthesized, and these synthesized peptides were purified using C18 reversephase high-performance liquid chromatography (HPLC) (Thermo Fisher Scientific, USA). The column used was ACQUITY UPLC BEH300 C18 (2.1 mm × 100 mm, 1.7 µm, Waters Co, USA).

**[Table 1]**

| SEQ ID NO: | Amino acid sequence of peptide 1 |
|---|---|
| 1 | KIWNLEPSRNGT |

The efficacy of the peptide having SEQ ID NO: 1 prepared above was evaluated through the following experiment.

### Experimental Example 1: Cell proliferation analysis

The effect of the peptide having SEQ ID NO: 1 prepared in Preparation Example 1 above on cell proliferation was evaluated.

NIH3T3 cells (mouse fibroblast) and HaCaT cells (human keratinocyte cell line) were seeded at a density of 1 × 10⁴ cells/well in a 96-well plate and cultured for 24 hours. The cells were washed once with serum-free DMEM medium. Different amounts of peptides were added to 200 µL of serum-free DMEM medium to prepare peptide medium solutions having concentrations of 1.6 µM, 8 µM, 40 µM, 200 µM, and 1000 µM, and the prepared peptide medium solutions were treated to the washed cells. Then, the treated cells were cultured in a CO₂ incubator at 37°C for 72 hours, washed twice with PBS, and MTT (Sigma, Cat. No.: M2003, USA) solution was dispensed into each well to a final concentration of 0.5 mg/mL. Then, after blocking light, the cells were cultured in a CO₂ incubator at 37°C for 4 hours. The absorbance was measured at 540 nm using a microplate reader (SpectraMax iD3, Molecular devices, USA).

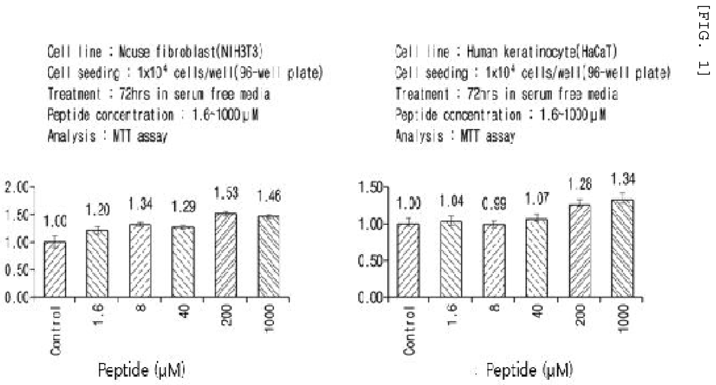

As a result of the experiment, as shown in the experimental results in FIG. 1, a significant effect of promoting cell proliferation was observed in both skin cell lines, NIH3T3 cells and HaCaT cells, when treated with peptides.

### Experimental Example 2: Analysis of promotion of collagen and hyaluronic acid (HA) expression

The effect of the peptide having SEQ ID NO: 1 prepared in Preparation Example 1 above on the expression of collagen and hyaluronic acid (HA) was confirmed.

NIH3T3 cells (mouse fibroblast) and HaCaT cells (human keratinocyte cell line) were seeded at a density of 1 × 10⁴ cells/well in a 24-well plate and cultured for 24 hours. The cells were washed once with serum-free DMEM medium. Different amounts of peptides were added to 500 µL of serum-free DMEM medium to prepare peptide medium solutions having concentrations of 50 µM, 100 µM, and 200 µM, and the washed cells were treated with the prepared peptide medium solutions. As a positive control, 100 nM TGF-β1 was used in NIH3T3 cells and 100 nM IGF-1 was used in HaCaT cells. The treated cells were then cultured in a CO₂ incubator at 37°C for 72 hours. The cell culture liquid was collected, centrifuged, and the supernatant was transferred to a tube. The obtained supernatant was subjected to ELISA analysis using the Collagen ELISA kit (Abcam, Cat No.: ab210579, UK) and HA ELISA kit (ECHELON Biosciences, Cat No.: K-1200, USA), respectively.

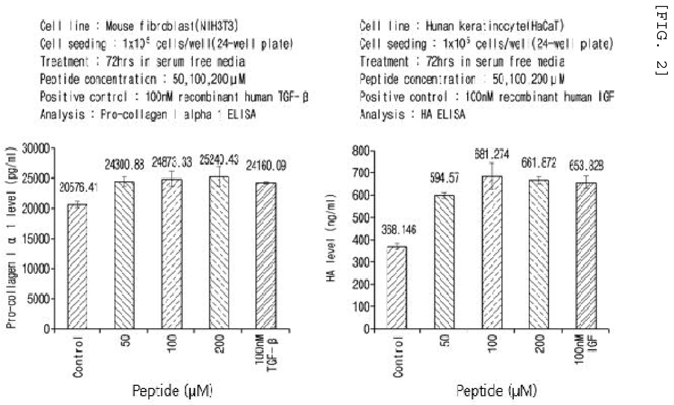

As a result of the experiment, as shown in the experimental results in FIG. 2, when the peptide was treated, a significant increase in collagen expression and an increase in hyaluronic acid (HA) expression were observed in both NIH3T3 cells and HaCaT cells at concentrations of 50 µM to 200 µM. These results suggest that the peptide of the present invention stimulates skin cells to thereby promote the production of extracellular matrix (ECM).

### Experimental Example 3: Analysis of effect of delaying degradation of hyaluronic acid (HA) by hyaluronidase

It was evaluated whether the peptide having SEQ ID NO: 1 prepared in Preparation Example 1 above has an effect of delaying the degradation of hyaluronic acid by hyaluronidase.

### 1. Evaluation of delay of hyaluronic acid degradation in hyaluronic acid solution

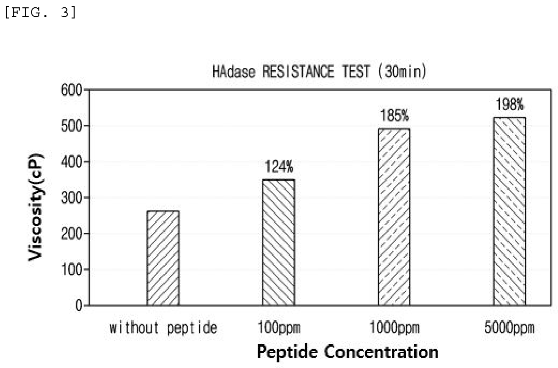

Hyaluronic acid was dissolved in PBS to a concentration of 1.5 wt% and hydrated, and 10 g of this solution was dispensed into each conical tube. Peptide solutions were added to the dispensed hyaluronic acid solutions so that the final peptide concentrations were 0 ppm (no addition), 100 ppm, 1000 ppm, and 5000 ppm, respectively. 2 mL of hyaluronic acid degrading enzyme (hyaluronidase from bovine testes, Hadase, Sigma) was added to each conical tube. Viscosity was measured using a rheometer (single shear stress) by sampling at 0 and 30 minutes to compare the viscosity values according to the peptide concentration. As above, the 1.5 wt% hyaluronic acid solution containing the peptides at each concentration was treated with hyaluronidase, and the enzymatic reaction was maintained for 30 minutes to decompose hyaluronic acid. After the degradation reaction, the viscosity of the hyaluronic acid solution was measured to confirm the degradation delay efficacy. As a result of the experiment, as shown in FIG. 3, it was confirmed that the degradation of hyaluronic acid was delayed as the concentration of the peptide increased. Since the difference in the degradation delay efficacy of 1000 ppm and 5000 ppm was not significant, the subsequent experiment was performed at a concentration of 1000 ppm.

### 2. Evaluation of delay of hyaluronic acid degradation in cross-linked fillers

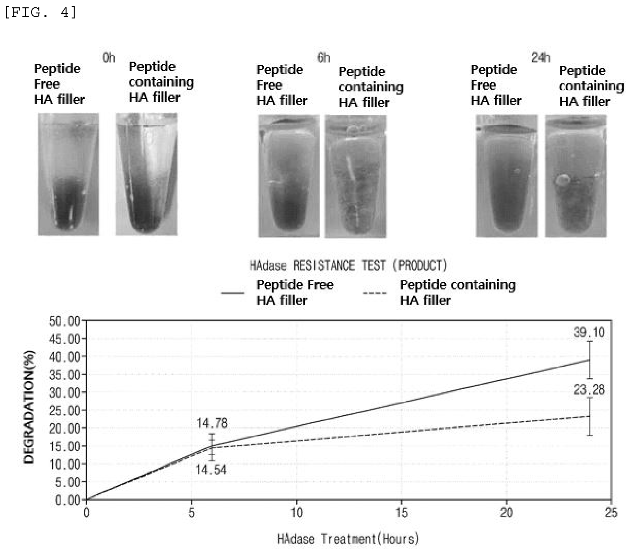

A 1,4-Butanediol diglycidyl ether (BDDE)-cross-linked hyaluronic acid filler with or without the peptide of Preparation Example 1 was prepared, and Congo Red and hyaluronidase (HAdase) were prepared as absorbing dyes. 20 µL of 1 mM Congo Red was added to a 1.5 mL eppendorf tube, and 100 µL each of the peptide-free filler (control filler) and the peptide-containing filler were added on the absorbing dye. Each was treated with 350 µL of 0.15 mg/mL (108 units) of hyaluronidase. Each tube was placed in an incubator at 37°C, and 100 µL of the sample supernatant was collected at time points of 6 and 24 hours and transferred to a 96-well. The absorbance was measured at 500 nm using a microplate reader (SpectraMax iD3, Molecular Devices, USA). As above, BDDE-cross-linked hyaluronic acid filler samples with or without the peptide were prepared, and the degree of degradation of the hyaluronic acid filler by hyaluronidase was compared depending on the presence or absence of the peptide. The degree of degradation of the hyaluronic acid filler was measured by the change in absorbance according to the diffusion of the dye. That is, the principle, in which the flowability of the sample increases when hyaluronic acid is degraded by the degradation enzyme, thereby increasing the diffusion of the dye, was utilized. As a result of the experiment, as shown in FIG. 4, it was confirmed that the delay of degradation was improved by 15% or more due to the peptide contained in the sample 24 hours after the treatment with hyaluronidase.

### Experimental Example 4: Analysis of effect of delaying hyaluronic acid (HA) degradation by reactive oxygen species

It was evaluated whether the peptide having SEQ ID NO: 1 prepared in Preparation Example 1 above has an effect of delaying the degradation of hyaluronic acid by reactive oxygen species.

### 1. Evaluation of delay of hyaluronic acid degradation in hyaluronic acid solution

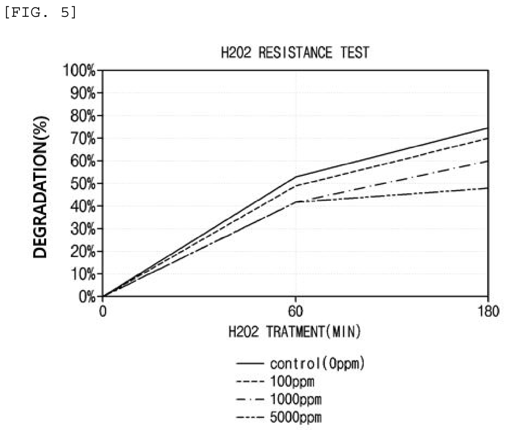

Hyaluronic acid was dissolved in PBS to a concentration of 1.5 wt% and hydrated, and 10 g of this solution was dispensed into each conical tube. Peptide solutions were added to the dispensed hyaluronic acid solutions so that the final peptide concentrations were 0 ppm (no addition), 100 ppm, 1000 ppm, and 5000 ppm, respectively. 2 mL of 500 mM hydrogen peroxide (H₂O₂) was added to each. Viscosity was measured using a rheometer (single shear stress) by sampling at 0, 30, 60, and 180 minutes to compare the viscosity values according to the peptide concentration. As above, 1.5 wt% hyaluronic acid solutions containing peptides at each concentration were treated with hydrogen peroxide to maintain the degradation reaction by reactive oxygen species for 60 and 180 minutes, thereby degrading hyaluronic acid. After the degradation reaction, the viscosity of each hyaluronic acid solution was measured to confirm the degradation delaying efficacy. As a result of the experiment, as shown in FIG. 5, it was confirmed that the degradation of hyaluronic acid was delayed as the concentration of peptide increased.

### 2. Evaluation of delay of hyaluronic acid degradation in cross-linked fillers

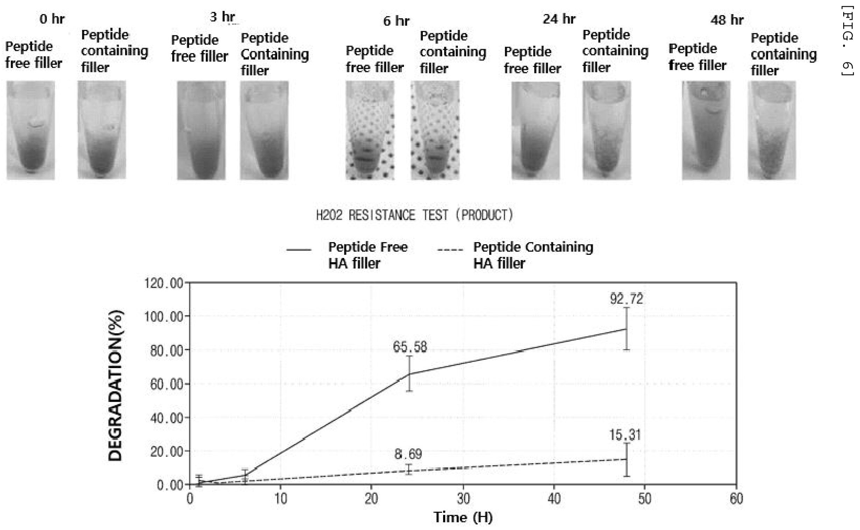

A 1,4-Butanediol diglycidyl ether (BDDE)-cross-linked hyaluronic acid filler with or without the peptide of Preparation Example 1 was prepared, and Congo Red and hyaluronidase (HAdase) as absorbing dyes were prepared. 20 µL of 1 mM Congo Red was added to each 1.5 mL eppendorf tube, and 100 µL each of the peptide-free filler (control filler) and the peptide-containing filler were added on the absorbing dye. Each was treated with 350 µL of 500 mM hydrogen peroxide (H₂O₂). Each tube was placed in an incubator at 37°C, and 100 µL of the sample supernatant was collected at time points of 1 hour, 3 hours, 6 hours, 24 hours, 48 hours, and 72 hours and transferred to 96-wells. The absorbance was measured at 500 nm using a microplate reader (SpectraMax iD3, Molecular devices, USA). As above, BDDE-cross-linked hyaluronic acid filler samples with or without the peptide were prepared, and the degree of degradation of hyaluronic acid filler by reactive oxygen species was compared depending on the presence or absence of peptide. The degree of degradation of hyaluronic acid filler was measured by the change in absorbance due to the diffusion of dye. That is, the principle that the flowability of the sample increases when hyaluronic acid is degraded by reactive oxygen species and thus the diffusion of the dye increases was utilized. As shown in FIG. 6, the experimental results showed that the degradation delay was improved by 50% or more due to the peptides contained in the sample 24 hours after the treatment with hydrogen peroxide (reactive oxygen species), and in the sample treated for 48 hours, most of the hyaluronic acid was degraded in the filler without peptides, whereas an effect of delaying degradation by 85% was confirmed when peptides were contained.

### Experimental Example 5: Evaluation of pH stability and thermal stability of peptides

The pH stability and thermal stability of the peptide having SEQ ID NO: 1 prepared in Preparation Example 1 above were evaluated.

The evaluation of pH stability was performed as follows. A diluted solution of the peptide of Preparation Example 1 at a concentration of 1000 ppm was prepared, and the initial pH value of the solution was measured (pH = 3.21). 0.5 M NaOH was added so as to titrate the pH of the solution to pH 4, pH 7, and pH 10, respectively. Each sample was filtered through a 0.22 µm filter. The area values were compared based on pH 7 measured by HPLC.

The evaluation of thermal stability was performed as follows. A diluted solution of the peptide of Preparation Example 1 at a concentration of 1000 ppm was prepared, dispensed into 50 mL conical tubes, and placed in an incubator at 25°C and 50°C. After 72 hours, 1 mL of each sample was filtered through a 0.22 µm filter. The area values were compared at 25°C based on pH 7 measured by HPLC.

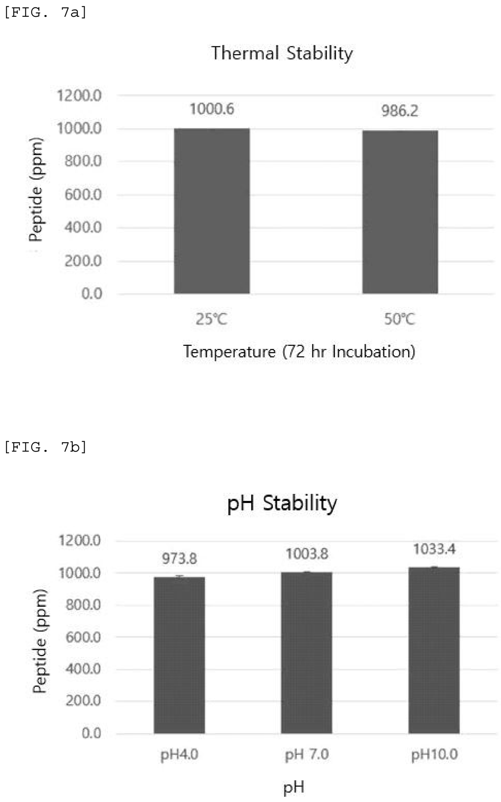

As a result of the experiment, as shown in FIGS. 7a and 7b, it was confirmed that the peptide of Preparation Example 1 was stable even at a high temperature of 50°C and in the range of pH 4 to pH 10.

Although the representative embodiments of the present application have been described above as examples, the scope of the present application is not limited to the specific embodiments described above, and those with ordinary knowledge in the relevant art will be able to make appropriate changes within the scope described in the claims of the present application.

## Claims

1. A peptide comprising an amino acid sequence of SEQ ID NO: 1.

2. The peptide of claim 1, wherein the peptide has an activity of delaying the degradation of hyaluronic acid.

3. The peptide of claim 1, wherein the peptide has an activity of promoting the proliferation of fibroblasts or keratinocytes; or promoting the expression of collagen or hyaluronic acid.

4. A composition for skin regeneration or skin condition improvement, comprising the peptide of claim 1 as an active ingredient.

5. A cosmetic composition for skin regeneration or skin condition improvement, comprising the peptide of claim 1 as an active ingredient.

6. The cosmetic composition for improving skin condition of claim 5, wherein the improvement of the skin condition is an improvement of skin elasticity, an improvement of skin wrinkles, an improvement of skin moisturization, inhibition of skin aging, a recovery of skin damage caused by ultraviolet rays, a protection of skin from ultraviolet rays, or an improvement of skin wounds.

7. A pharmaceutical composition for preventing or treating skin diseases, comprising the peptide of claim 1 as an active ingredient.

8. The pharmaceutical composition of claim 7, wherein the skin disease is a wound on the skin or scar on the skin.

9. A composition for delaying the degradation of hyaluronic acid, comprising the peptide of claim 1 as an active ingredient.

10. The pharmaceutical composition of claim 9, wherein the degradation of the hyaluronic acid is degradation by hyaluronidase or degradation by reactive oxygen species.

11. A filler composition comprising the peptide of claim 1 and hyaluronic acid.

12. The filler composition of claim 11, wherein the hyaluronic acid is cross-linked hyaluronic acid.

13. The filler composition of claim 12, wherein the cross-linked hyaluronic acid is cross-linked with one or more cross-linking agents selected from the group consisting of 1,4-butane diol diglycidyl ether (BDDE), polyethylene glycol (PEG), divinyl sulfone (DVS), poly (ethylene glycol) diglycidyl ether (PEGDGE), 1,6-hexanediol diglycidyl ether, propylene glycol diglycidyl ether, poly (propylene glycol) diglycidyl ether, and poly (tetramethylene glycol) diglycidyl ether.

14. The filler composition of claim 11, wherein the filler composition is used for improving wrinkles and tissue volume reduction of soft tissues selected from the group consisting of the neck, chest, buttocks, arms, armpits, hands, legs, and feet, or for treating wounds, scar, or stretch marks.
